# FASCICULE DE BREVET EUROPEEN

(11) **EP 2 555 659 B1**
(45) Date de publication et mention de la délivrance du brevet: **12.07.2017**
(21) Numéro de dépôt: 11765109.1
(22) Date de dépôt: 06.04.2011
(51) Int. Cl.: A61F 5/442, A61F 5/451

(54) **Système d'évacuation des fluides corporels**
System zur Ableitung von Körperflüssigkeiten
System for collecting body fluids

(30) Priorité: 08.04.2010 DZ 100198
(43) Date de publication de la demande: 13.02.2013
(73) Titulaire: Mokrane, Mohamed, Alger 16000 (DZ)
(72) Inventeur: Mokrane, Mohamed, Alger 16000 (DZ)
(74) Mandataire: Garavelli, Paolo
(86) Numéro de dépôt international: PCT/DZ2011/000002
(87) Numéro de publication internationale: WO 2011/124231

(56) Documents cités:
- EP-A1- 0 610 638
- EP-A1- 0 610 638
- EP-A2- 1 093 782
- WO-A1-00/33773
- WO-A1-2009/145602
- WO-A2-2006/014240
- FR-A- 1 485 683
- FR-A1- 2 905 593
- GB-A- 2 050 838
- US-A- 1 178 644
- US-A- 3 721 243
- US-A- 4 631 061
- US-A1- 2009 270 822

## Description

### Domaine technique auquel se rapporte l'invention :

Gestionnaire des liquides handicapants, consiste en un dispositif, pour la gestion complète des flux d'urine volontaire ou involontaire ainsi que le cycle des écoulements menstruels pour les femmes, dans la vie du quotidien même en mouvement de façon discrète, cet appareillage se veut autonome, hermétique, léger, confortable et discret, possède un système de lavage, de désinfection du pénis et du conduit après l'acte d'urine, doté d'une option sac-dos réservoir avec robinet télescopique, ce qui permet d'accomplir aussi l'acte rituel de purification de certaines parties du corps par l'eau en l'occurrence les ablutions des prières pour les musulmans.

### Etat de la technique antérieur:

On utilise pour la gestion de l'incontinence des couches en plastiques jetables absorbantes, dont certaines pour les femmes, possèdent une poudre pour geler l'urine et éviter son débordement, comme il existe un système appelé PENI-FLOW relié à un collecteur.

Différents systèmes d'évacuation des fluides corporels selon le préambule de la revendication 1 annexée sont connus dans l'art antérieur, comme par exemple ceux divulgués dans FR1485683, EP0610638, WO2006/014240 ou US4631061. Ces systèmes comportent une ou plusieurs pompes électriques, et, lorsqu'ils sont portables, sont incorporés de façon compacte au sein d'une ceinture-culotte, d'une genouillère ou d'un sac à dos respectivement.

### But de l'invention:

Le but de l'invention consiste à redonner une qualité de vie normale aux malades de part la parfaite gestion de l'incontinence et de l'écoulement menstruel des femmes, afin de les libérer le libérer complètement de cet handicap par une totale capacité de mouvement en toute aisance. Comme il permet aux musulmans une hygiène permanente, l'accomplissement des ablutions pour leurs prières. Et aux lieux saints de l'Islam à la Mecque les jours de pèlerinage du Hadj de stabiliser les mouvements ce qui permettra d'augmenter la surface donc le nombre de pèlerins à Mina et Djébel Arafat, d'éviter les contactes pour réduire les contagions épidémiques, d'augmenter la sécurité des personnes et de leur donner plus de repos. Ce but est atteint au moyen d'un système tel que défini dans la revendication 1 indépendante annexée. Différents modes de réalisation sont en outre définis dans les revendications dépendantes.

### ENONCE DES FIGURES :

**Figure n° 1 :**
   **Habit principal** :
   Il est constitué d'un maillot slip ou cuissard comportant :
      - Une ouverture pour la fixation de la frange de la coquille servant d'urinoir.
      - Des ouvertures latérales, pour pouvoir déféquer sans dé scotché le système, tenir la partie avant du maillot qui deviendra indépendante de la partie arrière avec réglage des tailles une ceinture pour le support de la pompe de lavage.
**Figure N° 2 :**
   **Coquille en latex :**
   Elle est constituée d'un plan incurvé ayant la forme d'une demie ellipsoïde (ayant subie une rotation autour du grand axe).
   Sur cette coquille sont greffes trois parties qui sont :
      a) La membrane bombée et flasque qui fait office d'urinoir en sa partie extérieur.
      b) Le manchon du haut (il est ouvert) permettant de recevoir le pénis.
      c) La poche du bas permettant de loger le scrotum.
   La position des parties b et c sera celle d'utilisation de la coquille après déchaussement du moule (en réalité dans le moule, ces deux parties seront inversées).
   Un orifice du haut est celui réservé au tube pénétrant dans la coquille. En réalité ce tube pénètre dans le moignon solidaire de la coquille. Il est cylindrique et ayant de 5 mm de hauteur et 5 mm de diamètre.
   Le tube du bas sortant de la coquille ce sera un orifice ayant la forme d'une pyramide inversée, il jouera le rôle d'entonnoir. Cette pyramide est constituée de deux cotés de formes trapézoïdales égales et symétriques.
   La partie surfacée plane elliptique face intérieur de la coquille, fermant et épousant la forme des ½ ellipsoïdes. Le prolongement de la partie surface représente la frange dépassant de 3 cm pour la fixation de la coquille à l'habit principal.
**Figure N°3 :**
   **Genouillère :**
   Elle jouera le rôle de porte collecteur.
**Figure N° 4 :**
   **Collecteur :**
   Sachet collecteur composé d'alvéoles en forme de bulles ou d'une demi-sphère coupée le long d'un diamètre, qui communiquent entre elles afin d'éviter l'effet « poche boule » en sa phase finale a la réception et le stockage des liquides. Hermétique souple et /ou élastique.
**Figure N° 5 :**
   **Réservoir :**
   pompe nouvelle de forme rectangulaire pour la contenance du liquide de lavage et de désinfection pour les femmes afin d'éviter un foyer microbien, munis d'un clapet trappe de sortie et une bouche de remplissage et d'une tubulure reliée à l'arrosoir de la coquille en sa partie supérieur. Elle fonctionne par pression manuelle et à la demande.
**Figure N°6 :**
   **a) Arrosoir :**
      il sera situé vers le haut de la coquille et disposé de telle sorte à envoyer le liquide avec un jet énergique.
   **b) Tubulures :**
      - La tubulure reliant la coquille au collecteur est plate à grand débit. Elle se trouve dans le prolongement de l'entonnoir qui termine la coquille.
      - La tubulure reliant la pompe à la coquille est classique de forme arrondie.
   **c) Clapets :**
      Ils sont au nombre de quatre, les deux premiers qui ont des tailles différentes ont des fonctions identiques.
      Clapet du collecteur, il comporte une trappe d'ouverture vers l'intérieur ou viendrait s'emboiter le clapet anti-retour de la tubulure plate d'évacuation venant de la coquille.
         - Clapet de la pompe qui sert à libérer le liquide pour le lavage ou la désinfection par pression manuelle (sur la pompe en caoutchouc tendre). Son rôle est de stopper le liquide ou de le libérer lors de la pression manuelle.
         - Clapet anti-retour, se situe au bout de la tubulure plate reliant la coquille au collecteur.
         - Clapet d'évacuation d'air, sa fonction est de libérer l'air tout en empêchant les liquides de passer pour faciliter le mouvement des liquides, et l'évacuation au collecteur. Il sera situé sur le niveau supérieur de la coquille à environ 10 mm du moignon cylindrique. Il est semblable à celui utilisé dans les aquariums.
   **L'adhésif :**
   L'adhésif qui sera utilisé est un hypoallergénique à double face, il servira à sceller et à fermer de façon hermétique l'urinoir, constitué par la coquille sur la peau.
   Il aura une largeur de 15 mm environ pour l'homme il épousera une forme elliptique de façon à passer autour du manchon du pénis et de la poche du scrotum.
   Pour la femme, à compter du bord de la coquille compter 15 mm de forme ovale autour de la partie génitale.
**Figure N° 7 :**
   **gilet des ablutions :**
   Il est composé d'une seule pièce en latex en tissu de forme rectangulaire, ou viendrait se greffés 2 poches réservoir d'eau avec 2 sangles de fixation et une ceinture, doté d'une tubulure télescopique de 40 cm, en son bout un robinet tige de 7 cm avec une fonction de fermeture et d'ouverture en demi-lune.
   **Présentation de l'invention :**
      L'invention consiste en un système qui permet de gérer d'une manière complète, l'handicap de l'incontinence, et l'écoulement menstruel des femmes, de donner une indépendance d'uriner en en tout lieu et à tout moment, même en mouvement. La nouveauté de ce système réside dans le fait que l'appareil est constitué d'un ensemble d'éléments inconnus à ce jour, lui donnant une capacité totale de gestion des urines, et des écoulements menstruels.
      Il s'agit des éléments suivants :
         - Un habit maillot, doté d'une coquille urinoir avec un emplacement pour le pénis et le scrotum.
         - Une genouillère à poches pour le support des collecteurs alvéoles pour éviter l'effet « boule »
         - Une pompe manuelle, réservoir des liquides, pour le lavage et la désinfection.
         - Le collecteur, sans effet de boule, est compartimenté en alvéoles, et présente une grande capacité de stockage, des tubulures, et des clapets ayants différentes fonctions.
         - Un gilet sac-dos, doté de sangles et d'une ceinture de fixation. Ce gilet est conçu de façon à stocker de l'eau destinée aux ablutions. Il est muni d'une tubulure métallique télescopique avec robinet conçu en demi-lune pouvant s-ouvrir et se fermer d'une façon souple et pratique.

### Mode de réalisation de l'invention

La réalisation de l'invention consiste par procédé de moulage des pièces suivantes :
- Coquille urinoir porte pénis et scrotum en latex par technologie de trempage.
- Moulage de la pompe manuelle en caoutchouc tendre par technologie d'injection ou presse.
- Moulage du collecteur alvéolé par technologie d'injection ou presse en plastique.
- confection de la genouillère en latex fin ou tissus résistant élastiqué.
- Tubulures en latex plate à grand débit.
- Clapets et adhésif anti-allergénique.
- Gilet des ablutions confectionné en latex fin avec des sangles et sa ceinture, fabrication d'une tubulure télescopique de type nouveau.

### Manière dont l'invention est susceptible d'application:

Cette invention concerne les deux sexes, adolescents et adultes. L'utilisation commence par placer l'habit comme une culotte et scotcher le pourtour de la coquille autour du pénis et du scrotum par un adhésif à double face anti-allergénique et médical, ensuite régler selon les mensurations par les ouvertures latérales. Placer la pompe réservoir au niveau de la hanche gauche dans la ceinture de fixation et raccorder sa tubulure de lavage.

Fixer la genouillère au dessus du genou jusqu'au niveau de la cheville. Prendre le collecteur alvéolé, le raccorder à la tubulure du flux muni d'un clapet anti-retour et le loger dans la poche de la genouillère.

### Description textuelle -règle de forme :

Le patient s'habille comme ou porte un slip.

Règle le dispositif selon sa taille par les ceintures des ouvertures latérales.

Place le pénis dans le manchon de la coquille urinoir et loge le scrotum pour les hommes, procède à l'hermetisation en scotchant la coquille à la peau avec l'adhésif double face.

Ensuite il installe la genouillère, fixe la pompe, branche le collecteur à l'embout de la tubulure.

Quand l'utilisateur urine, le flux est géré de manière hermétique, tout d'abord le pénis déverse l'urine dans la coquille urinoir, de cette dernière, elle n'a que le choix du passage par la tubulure le long de la cuisse intérieure pour arriver et se déverser dans le collecteur. Pour les femmes l'écoulement est gérer identiquement

Ensuite le malade actionne la pompe manuellement par pression qui agit sur le clapet d'ouverture et libère le liquide sous pression, distribué par l'arrosoir, pour un lavage efficace et une désinfection de part l'antiseptique que contient l'eau. Le cheminement est identique et les conduites du dispositif sont lavés et désinfectés au même titre que les parties génitales.

Le collecteur d'une grande capacité de part sa conception, une fois rempli on le détache de la tubulure, son clapet automatiquement se ferme et le sachet est hermétiquement clos pour être jeté dans une poubelle sans risque de détérioration. Il est rapidement remplacé par un sachet de réserve logé dans le magasin de la genouillère.

Les ouvertures latérales du slip permettent aux patients de déféquer sans dé scotché le procédé de fixation de la coquille, et de régler les mensurations.

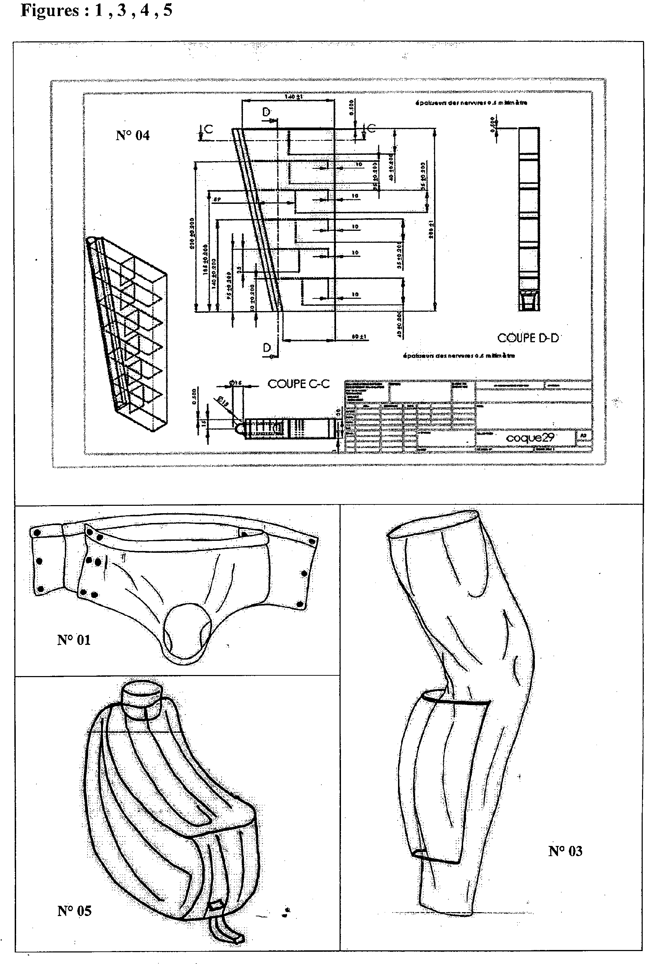

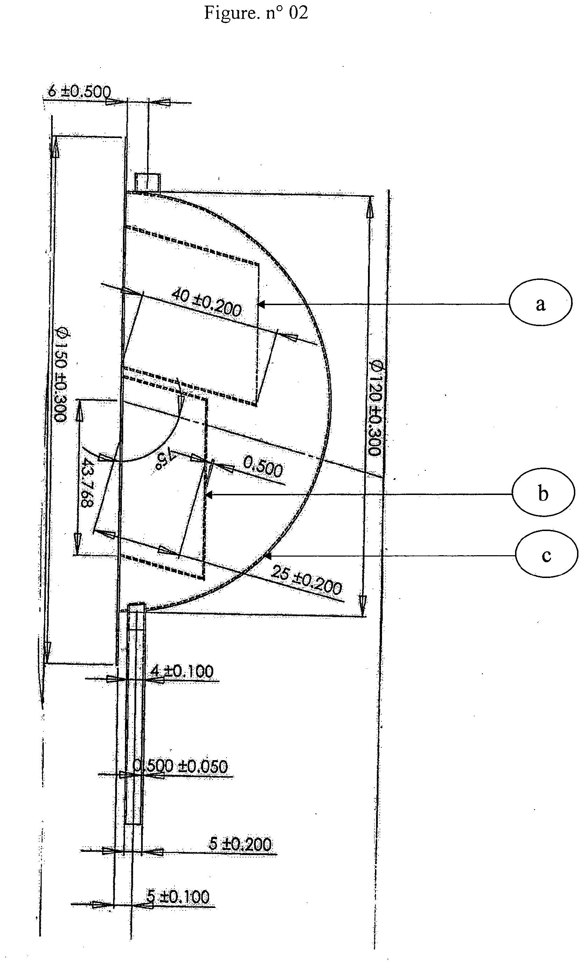

## Revendications

1. Système d'évacuation des fluides corporels, à savoir l'urine ou les écoulements menstruels comprenant :
un slip accueillant une coquille,
un collecteur de fluide relié à ladite coquille par une tubulure,
un réservoir de liquide de lavage ou de désinfection, une pompe pour amener ledit liquide de lavage reliée à ladite coquille par une tubulure; **caractérisé en ce que** ledit réservoir est configuré pour être porté sur le dos ;
ledit collecteur de fluide est un collecteur alvéolé configuré pour être porté au moyen d'une genouillère ;
ladite pompe est une pompe à actionnement manuel.

2. Système selon la revendication 1 **caractérisé en ce que** ledit réservoir comprend un gilet sac-à-dos apte à stocker le liquide de lavage ou de désinfection.

3. Système selon la revendication 1 ou 2 caractérisé en que ladite coquille est pourvue d'un arrosoir de liquide.

4. Système selon l'une des revendications 1 à 3 **caractérisé en ce que** ladite coquille comprend un entonnoir recueillant ledit fluide.

5. Système selon l'une quelconque des revendications 1 à 4 **caractérisé en ce que** lesdites alvéoles dudit collecteur communiquent entre elles.

6. Système selon l'une quelconque des revendications 1 à 5 **caractérisé en ce que** ladite tubulure reliant ladite coquille audit collecteur est une tubulure plate à haut débit.

7. Système selon l'une des revendications 1 à 6 **caractérisé en ce que** ladite tubulure reliant ladite coquille audit collecteur est équipée d'un clapet anti-retour.

8. Système selon l'une quelconque des revendications 1 à 7 **caractérisé en ce que** ladite
pompe est
équipée
d'un clapet stoppant ou libérant ledit liquide de lavage ou de désinfection par pression manuelle sur ladite pompe.

9. Système selon l'une quelconque des revendications 1 à 8 **caractérisé en ce que** la partie supérieure de ladite coquille comprend un clapet d'évacuation d'air.

10. Système selon l'une quelconque des revendications 2 à 9 **caractérisé en ce que** ledit gilet sac-à-dos est muni d'un tubulure métallique télescopique.

## Patentansprüche

1. Abflusssystem von Körperflüssigkeiten, d. h. Harn oder Menstruationsflüssigkeiten, das Folgendes enthält:
eine Unterhose, die eine Schale enthält,
einen Flüssigkeitssammler, der mit der genannten Schale durch einen Schlauch verbunden ist,
einen Wasch- oder Desinfektionsflüssigkeitsbehälter,
eine Pumpe, um die genannte Waschflüssigkeit zu leiten, die mit der genannten Schale durch einen Schlauch verbunden ist;
und **dadurch gekennzeichnet ist, dass**:
der genannte Behälter dazu konfiguriert ist, auf dem Rücken getragen zu werden;
der genannte Flüssigkeitssammler ein wabenförmiger Sammler ist, der konfiguriert ist, um durch einen Knieschützer getragen zu werden;
die genannte Pumpe eine Pumpe mit Handantrieb ist.

2. System gemäß Patentanspruch 1, das **dadurch gekennzeichnet ist, dass** der genannte Behälter eine Rucksackweste enthält, um die Wasch- oder Desinfektionsflüssigkeit zu speichern.

3. System gemäß Patentanspruch 1 oder 2, das **dadurch gekennzeichnet ist, dass** die genannte Schale mit einem Flüssigkeitsausgießer ausgestattet ist.

4. System gemäß einem der Patentansprüche von 1 bis 3, das **dadurch gekennzeichnet ist, dass** die genannte Schale einen Trichter enthält, der die genannte Flüssigkeit aufnimmt.

5. System gemäß einem der Patentansprüche von 1 bis 4, das **dadurch gekennzeichnet ist, dass** die genannten Waben des genannten Sammlers miteinander verbunden sind.

6. System gemäß einem der Patentansprüche von 1 bis 5, das **dadurch gekennzeichnet ist, dass** der genannte Verbindungsschlauch der genannten Schale mit dem genannten Sammler ein Flachschlauch mit hohem Durchfluss ist.

7. System gemäß einem der Patentansprüche von 1 bis 6, das **dadurch gekennzeichnet ist, dass** der genannte Verbindungsschlauch der genannten Schale mit dem genannten Sammler mit einem Rückschlagventil ausgestattet ist.

8. System gemäß einem der Patentansprüche von 1 bis 7, das **dadurch gekennzeichnet ist, dass** die genannte Pumpe mit einem Ventil ausgestattet ist, das die genannte Wasch- oder Desinfektionsflüssigkeit durch einen Druck mit der Hand auf die genannte Pumpe anhält oder freisetzt.

9. System gemäß einem der Patentansprüche von 1 bis 8, das **dadurch gekennzeichnet ist, dass** der genannte obere Teil der genannten Schale ein Luftablassventil enthält.

10. System gemäß einem der Patentansprüche von 2 bis 9, das **dadurch gekennzeichnet ist, dass** die genannte Rucksackweste mit einem Teleskopschlauch aus Metall ausgestattet ist.

## Claims

1. System for evacuating body fluids, namely urine or mestrual fluxes, comprising:
a pair of underpants housing a shell,
a fluid collector connected to said shell through a piping,
a tank for washing or disinfecting liquid,
a pump to take said washing liquid connected to said shell through a piping;
**characterized in that**
said tank is configured to be carried on a back;
said fluid collector is a recessed collector configured to be carried by means of a toggle joint;
said pump is a manually actuated pump.

2. System according to claim 1, **characterized in that** said tank comprises a backpack gilet adapted to store the washing or disinfecting liquid.

3. System according to claim 1 or 2, **characterized in that** said shell is equipped with a liquid spraying device.

4. System according to an one of claims 1 to 3, **characterized in that** said shell comprises a funnel for collecting said fluid.

5. System according to an one of claims 1 to 4, **characterized in that** said recesses of said collector are mutually communicating.

6. System according to an one of claims 1 to 5, **characterized in that** said piping connecting said shell to said collector is a plate piping with high flow-rate.

7. System according to an one of claims 1 to 6, **characterized in that** said piping connecting said shell to said collector is equipped with a non-return valve.

8. System according to an one of claims 1 to 7, **characterized in that** said pump is equipped with a valve which stops or releases said washing or disinfecting liquid through a manual pressure on said pump.

9. System according to an one of claims 1 to 8, **characterized in that** the upper part of said shell comprises an air exhaust valve.

10. System according to an one of claims 2 to 9, **characterized in that** said backpack gilet is equipped with a telescopic metallic piping.
